# EUROPEAN PATENT APPLICATION

(11) **EP 2 327 703 A1**
(43) Date of publication of application: **01.06.2011**
(21) Application number: 09810284.1
(22) Date of filing: 03.08.2009
(51) Int. Cl.: C07D 471/02, A61K 31/437, A61P 25/28

(54) **LIGAND WITH A BROAD SPECTRUM OF PHARMACOLOGICAL ACTIVITY, A PHARMACEUTICAL COMPOSITION, A MEDICINAL AGENT AND A METHOD OF TREATMENT**

(30) Priority: 22.08.2008 RU 2008134309
(71) Applicant: IVASHCHENKO, Andrey Alexandrovich, Moscow 127576 (RU); Alla Chem, LLC., Carson City, NV 89701 (US)
(72) Inventor: IVASHCHENKO, Alexander Vasilievich, Encinitas California 92024 (US); SAVCHUK, Nikolay Filippovich, Moscow 117420 (RU)
(74) Representative: Henrion, Oliver
(86) International application number: PCT/RU2009/000381
(87) International publication number: WO 2010/024717

(57) **Abstract**

The present invention is directed to the novel ligand with wide range of pharmacological activity including activity to GPCR receptors, ion channels and monoamine transporters representing 3-methyl-9-benzyl-1,2,3,4-tetrahydrocarboline naphthalene-1,5-disulfonate of formula **1**

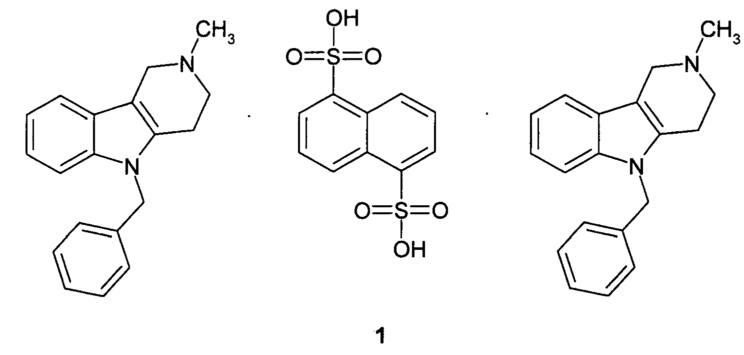

The invention is also directed to the novel drug substance, Pharmaceutical formulation comprising the novel drug substance, and to the method for its preparation, to the novel medicaments, and method for treatment of CNS diseases including neurodegenerative diseases and cognitive disorders at humans and warm blooded animals.

## Description

### Field of the invention

The invention is directed to the novel ligand with wide range of pharmacological activity, among them to GPCR receptors, ion channels and monoamine transporters, to novel active ingredient, Pharmaceutical formulation, comprising the said active ingredient, and to the method of its preparation, to novel medicaments, comprising the novel active ingredient, to therapeutic cocktail, and method for treatment of central nervous system (CNS) diseases including neurodegenerative diseases (ND) and cognitive disorders (CD) at humans and warm blooded animals.

### Background of the invention

The development of effective drugs for treatment of CNS diseases, among them ND and CD, has being conducted intensively for many years. A variety of medicaments and drug candidates for ND and CD treatment are known, the mechanism of action of which is associated with their ability to interact with one or more receptors, among them: adrenoceptors, dopamine receptors, histamine receptors, serotonin receptors and others. The role of adrenergic receptors has been established for many diseases of CNS, such as mental disorders, migraines, sleep disorders, nervousness, schizophrenia and so on [C.J. Zheng, L.Y. Han, C.W. Yap, Z.L.Ji, Z.W. Cao and Y.Z. Chen, Therapeutic Targets: Progress of Their Exploration and Investigation of Their Characteristics. Pharmacol Rev. 58:259-279, 2006]. Dopamine receptors play a key role in many processes of CNS, including the motivation control, training, locomotor functions, and also in modulation of neuroendocrine signaling. Dopamine receptors are the recognized neurological targets for psychotropic drugs, such as antipsychotic agents (receptor antagonists) and psychostimulants [Girault J., Greengard P. (2004). "The neurobiology of dopamine signaling". Arch Neurol. 61 (5): 641-4].

A great number of ligands (antagonists or/and agonists) with wide range of receptor-specific activity including interaction with α-adrenoceptors are known, as well as medicaments on their basis and drug candidates for treatment of various diseases and conditions of CNS. It makes possible to use such medicaments in wide range of therapeutic action. For example, (3aR,10cR)-2-methyl-1,2,3,3a,4,5,6,10c-octahydropyrrolo[3,4-c]carbazole **A** is α_{1A}-adrenoceptor, α_{1B}-adrenoceptor, α_{1D}-adrenoceptor antagonist, and α₂-adrenoceptor agonist. This compound is a non-opioid analgetic [WO 1999065911].

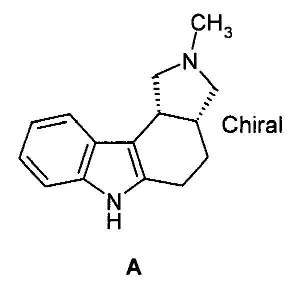

Central α₂-adrenoceptor antagonists stimulate noradrenaline release by blocking presynaptic α₂-receptors, which answer for negative control of this neurotransmitter release. Owing to the ability to increase noradrenaline concentration, α₂-antagonists could be used for prophylaxis or treatment or depression. They are potentially useful for treatment of Alzheimer's disease and mnestic disorders, because it is known that α₂-antagonists promote acetylcholine release [Tellez, et al., J. Neurochem. 1997, 68, 778-785].

The spectrum of Talipexole dihydrochloride **B** receptor activity, which is in the market since 1996 as a remedy for treatment of Parkinson's disease (PD), in addition to agonistic activity to dopamine D₂ receptors and dopamine autoreceptors, include agonistic activity to α₂-adrenoceptors [Boehringer Ingelheim, US 3804849].

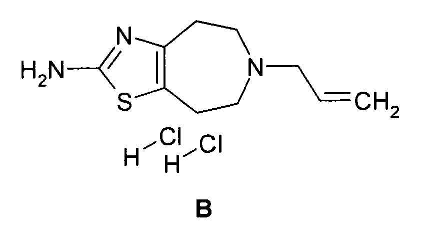

The spectrum of Pardoprunox **C** receptor activity, which is in the III phase of clinical trial as a remedy for PD treatment, together with agonistic activity to 5-HT_{1A} receptors and partial agonistic activity to dopamine D₂ receptors, involve agonistic activity to α₁-adrenoceptors and antagonistic activity to α₂-adrenoceptors [Solvay, WO 2005107754].

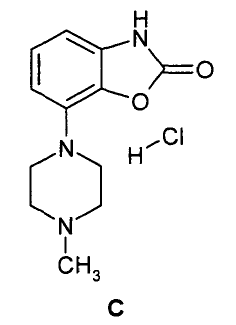

The development of effective medicaments for treatment of conditions and diseases of CNS and other neurodegenerative diseases has been conducted for many years. One of approaches to the solution of the problem is utilization of (antagonists or/and agonists) dopamine (D) receptor ligands, for example, D₁ receptor agonists [Sandoz Patent DE 3402392 - a remedy for PD treatment; Darpharma INC Patent, WO 2006012640 - antipsychotic agent, remedy for PD treatment and others; Smithkline Beecham PLC Patent, WO 2007022173 - remedy for hypertension treatment], D₁ receptor antagonist [Patent Novo Nordic AU 8936359 - remedy for sleep disorder treatment, alcohol dependency and nicotine addiction; antipsychotic drug], D₂ receptor agonists [Smithkline Beecham PLC Patent, WO 1996039136 - antidepressant, remedy for PD treatment and others; Nastech Pharmaceutical Company Patent WO 2002024202 - remedy for PD treatment, remedy for treatment of female sexual disorder and others].

A great number of ligands with wide range of receptor-specific activity are known. For example, D₁ - and D₂ - receptor agonists [Patent Maruk and others JP 1988033377 - remedy for PD treatment], D₁ receptor agonists and D₂ receptor antagonists [Patent Shanghainese institute of medicinal materials CN 1603324 - antipsychotic drug], D₁ - and D₂ -receptor antagonists [Schering Corporation Patent WO 1999044615 - remedy for obesity, alcohol and cocaine dependency treatment].

Ligands are known, exhibiting pronounced activity towards dopamine receptors in combination with affinity to serotonin 5-HT receptors, which are promising in medicaments for therapy of depressions, qualms and psychosis [Janssen Pharmaceutical Patent, US 6506768 B2, 2003].

Ligands with wide range of receptor-specific activity are used for preparation of medicaments with wide spectrum of therapeutic action. Table 1 represents some examples of drug candidates and medicaments for treatment of various conditions and diseases of CNS, the mechanism of action of which is determined by their wide range of receptor activity.

**Table 1. Certain ligands with wide range of receptor-specific activity.**

| **Formula, name** | **Patent** | **Therapeutic group** | **Phase** | **Mechanism of action** |
|---|---|---|---|---|
| 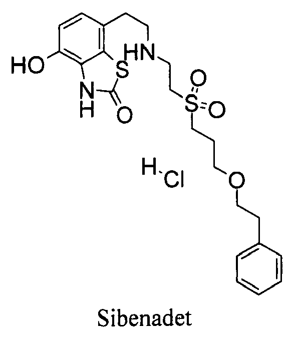 | strazeneca WO 006031181 | Bronchodilator | Phase III | D₂ receptor agonist, β₂-adrenoceptor agonist |
| 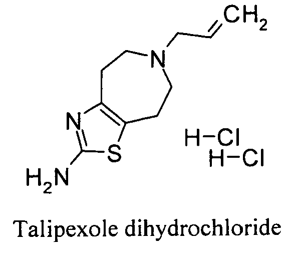 | boehringer ngelheim S 3804849 | PD treatment | In the market since 1996 | D₂ receptor agonist, α₂-adrenoceptor agonist, Dopamine autoreceptor agonist |
| 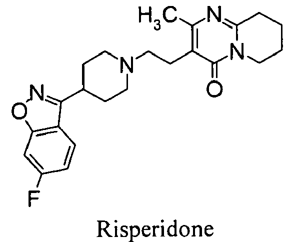 | Janssen WO 000023057 | Autism treatment, antidepressant drug, antipsychotic and others. | In the market since 1993 | D₂ receptor antagonist, 5-HT_{2A} receptor antagonist |
| 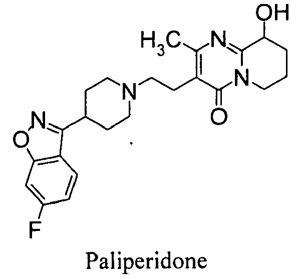 | Janssen WO 2007044234 | Antipsychotic | In the market since 1997 | D₂ receptor antagonist, 5-HT₂ receptor antagonist |
| 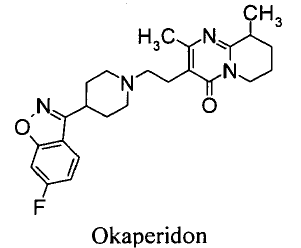 | Janssen Pharmaceutic EP 0196132, EP 0453042 | Antipsychotic | Phase II | D₂ receptor antagonist, 5-HT_{2A} receptor antagonist |
| 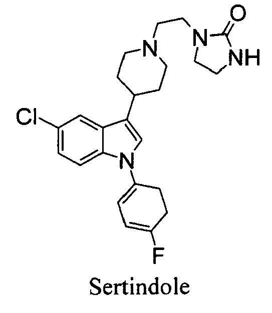 | Shionogi, WO 1997039752 | Antipsychotic | In the market since 2006 | D₂ receptor antagonist, 5-HT_{2A} receptor antagonist |
| 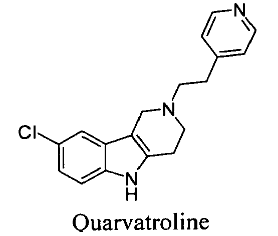 | American Home Products Corporation US 4636563 | Antipsychotic | Phase 1 | D₂ receptor antagonist, 5-HT_{2A} receptor antagonist |
| 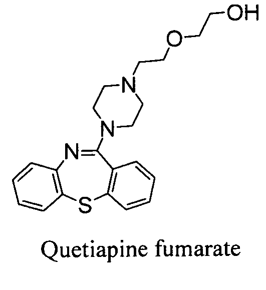 | Astrazeneca WO 2002062346 | Alcohol dependence, sleep disorder, psyphotic disorders and others | In the market since 1997 | D₂ receptor antagonist, 5-HT_{2A} receptor antagonist |
| 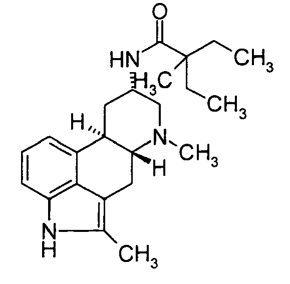 | Sandoz GB 2206115 | Antipsychotic | Phase II | Partial D₂ receptor agonist, D₁ receptor antagonist, partial 5-HT_{1A} receptor agonist |
| 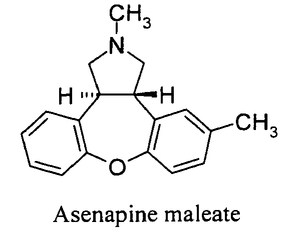 | Organon, Pfizer WO 2006040314 | Antipsychotic, bipolar illness | Phase III | D₁ receptor antagonist, D₂ receptor antagonist, 5-HT₂ receptor antagonist |
| 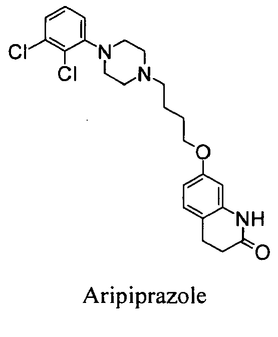 | Otsuka and others WO 2004060374 | Autism treatment, antidepressant drug, Alcohol dependence, bipolar illness, Cocaine dependence, Antipsychotic | In the market since 2002 | Partial D₂ receptor agonist, 5-HT_{1A} receptor agonist, 5-HT₂ receptor antagonist |
| 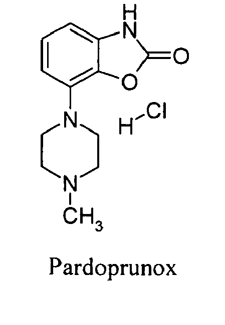 | Solvay Pharmaceutic WO 2005107754 | PD treatment | Phase III | Partial D₂ receptor agonist, 5-HT_{1A} receptor agonist, α₂-adrenoceptor antagonist, α₁-adrenoceptor agonist |
| 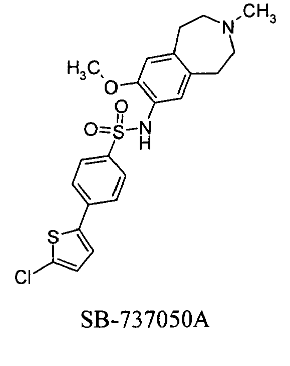 | Glaxo group WO 2003068752 | Antipsychotic | Phase II | D₂ receptor antagonist, D₃ receptor antagonist, 5-HT₆ receptor antagonist, 5-HT_{2A} receptor antagonist, 5-HT_{2C} receptor antagonist |
| 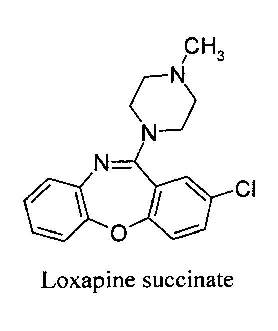 | White Pharmaceutical WO 2005023243 | Migraine, antipsychotic antidepressant drug | In the market since 1975 | D₂ receptor antagonist, D₁ receptor ligand, D₃ receptor ligand, D₄ receptor ligand, 5-HT₂ receptor antagonist |

One of the most perspective approaches to Alzheimer's disease (AD) and other neurodegenerative diseases treatment is based on the usage of effective antagonists active in relation to serotonin 5-HT₆ to receptors [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. At mammals these receptors are localized exclusively in CNS, and mainly in parts of brain responsible for training and memory [Ge'rard C., Martres M.-P., Lefe'vre K., Miquel M.-C., Verge' D., Lanfumey L., Doucet E., Hamon M., El Mestikawy S. Immuno-localisation of serotonin 5-HT6 receptor-like material in the rat central nervous system. Brain Research. 1997; 746:207-219]. Besides, it was shown, that 5-HT₆ receptors are modulators of the whole number of neuromediator systems, including cholinergic, noradrenergic, glutamatergic and dopaminergic [Dawson L.A., Nguyen H.Q., Li P. The 5-HT6 receptor antagonist SB-271046 selectively enhances excitatory neurotransmission in the rat frontal cortex and hippocampus. Neuropsychopharmacology. 2001; 25:662-668]. Taking into account the fundamental role of these systems in normal cognitive processes and also their dysfunction at neurodegeneration, exclusive role of 5-HT₆ receptors in forming normal and "pathological" memory becomes obvious. In a large number of nowadays publications it was shown that blocking of 5-HT₆ receptors leads to considerable enhancement of memory consolidation in various animal models of training-memorizing-reproduction [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100. Riemer C., Borroni E., Levet-Trafit B., Martin J.R., Poli S., Porter R.H., Bos M. Influence of the 5-HT6 receptor on acetylcholine release in the cortex: pharmacological characterization of 4-(2-bromo-6-pyrrolidin-1-yl-pyridine-4-sulfonyl)phenylamine, a potent and selective 5-HT6 receptor antagonist. J. Med. Chem. 2003; 46:1273-1276. King M.V., Woolley M.L., Topham I.A., Sleight A.J., Marsden C.A., Fone K.C. 5-HT6 receptor antagonists reverse delay-dependent deficits in novel object discrimination by enhancing consolidation an effect sensitive to NMDA receptor antagonism. Neuropharmacology 2004; 47:195-204]. It was also demonstrated that considerable enhancement of cognitive functions in aged rats took place under the influence of 5-HT₆ receptor antagonists in Morrison's water maze experiment [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100]. Recently more thorough understanding of 5-HT₆ receptor function in cognitive processes and more accurate conceptions concerning possible pharmacophoric properties of their antagonists were achieved. [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. This resulted in preparation of highly affine selective ligands ("molecular tools"), and afterwards clinical candidates. At present a considerable number of 5-HT₆ receptor antagonists are at various phases of clinical trial as potential active ingredients for treatment of various CNS diseases [http://integrity.prous.com].

The basis of pharmacological effect of antiallergic medicaments is their ability to reduce effectively cytosolic concentration of calcium when intracellular level of Ca⁺² ions has become excessive as a result of various pathological processes. Antihistaminic medicaments, in particular Mebhydrolin or Diazolin, fall into this class of compounds [Mashkovsky M.D. Pharmaceutical products. Pub. 13. Kharkov: Torsing, **1998.** v.1, p. 280].

Diazolin - 3-methyl-9-benzyl-1,2,3,4-tetrahydrocarboline naphthalene-1,5-disulfonate is a ligand (blocker) of H₁-receptors exhibits antiallergic action, reduces mucous coat edema. As an antiallergic medicament Diazolin is in the market for over 60 years [Horlein, U.; Hecht, G. Med. u. Chem., Abhandl. med.-chem. Forschungsstatten Farbenfabriken Bayer (1956), 5, 267-280. Charkevich, D.A. Pharm. And Toxicol., (1957), 20(6), 46-51]. Usage of Diazolin for treatment of CNS diseases among them ND and CD is not known.

The invention is directed to the novel ligand the range of pharmacological activity of which includes GPCR receptors, ion channels and monoamine transporters. The subject of the present invention is also an active ingredient (drug substance), Pharmaceutical formulation and method for its preparation, medicament and method for prophylaxis and treatment of various CNS diseases, among them ND and CD.

### Disclosure of the invention

In the context of the invention, the terms are generally defined as follows:
**"Agonists"** mean ligands being bound to the receptors of definite type actively promote transferring their specific signal and by that, cause the biological response of the cell.
**"Anxiolytic"** or **"Tranquilizer"** means a medicament intended for treatment of anxious disorders.
**"Antagonists"** mean ligands being bound to the definite receptors do not cause active cellular responses. Antagonists prevent linkage between agonists and receptors and by that block specific receptor signal transmission.
**"Antidepressant"** means a medicament intended for depression treatment.
**"Antipsychotic"** means a remedy intended for treatment of psychotic diseases.
**"Addictive substances"** mean medicaments, narcotics, psychoactive and physiologically active compounds, capable to cause addiction and dependence, such as alcohol, nicotine, amphetamines or sympathomimetics similar in their action; caffeine and its analogs, cannabinoids, cocaine and its analogs, hallucinogens, inhaled compounds, opiates, phencyclidine and its analogs, muscle pills, hypnotic and sedative drugs, and also any other natural, semisynthetic, synthetic or biotechnological compounds or mixtures of them capable to cause addiction and dependence.
**"Depression"** means big depression; incidental, chronic and recurring form of big depression; dysthymic disorder (dysthymia); cyclotymia; affective disorder; syndrome of seasonal affective disorder; bipolar disorder, including bipolar disorders of I and II type; and other depressive disorders and conditions. Depression also means the depressions accompanying AD, vascular dementia; disorder of mood induced by alcohol and substances; schizoaffective disorder of depressive type; disorder of adaptation. Except for that, depression includes depression of oncological patients; depression at Parkinson's disease; depressions after myocardial infarction; depressions of fruitless women; pediatric depression; postnatal depression; the depressions accompanying somatic, neuralgic and other diseases
**"Cognitive disorders or disorders of cognitive functions"** mean disorders (weakening) of mental abilities including attentiveness, memory, mentality, cognition, education, verbal, mental, executive and creative abilities, time and space orientation; in particular, cognitive disorders associated with AD, Parkinson's and Huntington's diseases, senile dementia; age-associated memory impairment, dysmetabolic encephalopathy; psychogenous memory impairment; amnesia; amnesic disturbances; transit global amnesia; dissociative amnesia; vascular dementia; light or mild cognitive impairment; attention deficit hyperactivity disorder (AD/HD); cognitive impairments, accompanying psychotic diseases, epilepsy, delirium, autism, psychosis, Down's syndrome, bipolar disorders and depression; AIDS-associated dementia; dementias at hypothyroidism; dementia connected with alcohol, substances causing dependability and neurotoxins; dementia accompanying neurodegenerative diseases, for example, cerebella degeneracy and amyotrophic lateral sclerosis; cognitive disturbances connected with cerebral crisis, infectious and oncological brain diseases as well as traumatic brain injury; cognitive function damages associated with autoimmune and endocrine diseases, and others.
**"Drug substance"** means physiologically active compound of synthetic or other (biotechnological, vegetable, animal, microbe and so on) origin exhibiting pharmacological activity and being an active ingredient of Pharmaceutical formulation employed in preparation and production of medicaments.
**"Medicament"** - is a compound or a mixture of compounds representing a Pharmaceutical formulation in the form of tablets, capsules, injections, ointments and other drug products intended for restoration, improvement or modification of physiological functions at humans and animals, and for prophylaxis and treatment of diseases, diagnostics, anesthesia, contraception, cosmetology and others.
**"Ligands"** (from Latin *ligo*) represent chemical compounds (small molecule, peptide, protein, inorganic ion, and so on) capable to interact with receptors which convert this interaction into specific signal. Ligands could be used for experimental investigation of physiological processes *in vivo* and *in vitro* as "pharmacological tools" or as a drug substance.
**"Neurodegenerative diseases"** means specific conditions and diseases, accompanied by damage and primary destruction of nervous cell populations in certain areas of CNS. Neurodegenerative diseases include but are not limited by: AB; Parkinson's and Huntington's (chorea) diseases; multiocular sclerosis; cerebella degeneracy; amyotrophic lateral sclerosis; dementias with Lewy bodies; spinal muscular atrophy; peripherical neuropathy; spongy encephalitis (Creutzfeld-Jakob Disease); AIDS dementia; multi-infarct dementia; frontotemporal dementias; leukoencephalopathy (spongy degeneration of white matter); chronic neurodegenerative diseases; cerebral crisis; ischemic, reperfusion and hypoxic brain damage; epilepsy; cerebral ischemia; glaucoma; traumatic brain injury; Down's syndrome; encephalomyelitis; meningitis; encephalitis; neuroblastoma; schizophrenia; depression. Moreover, neurodegenerative diseases include pathological states and disorders associated with hypoxia, substance abuse, causing dependability, under neurotoxins influence; infectious and oncological brain diseases as well as neuronal damages associated with autoimmune and endocrine diseases and others.
**"Nootrops"** or **"Nootropics"** (neurometabolic stimulates) are medicaments taken for cognition enhancing.
**"Substance-related disorder"** means substance use disorders, such as, substance dependence for example, drug substances, alcohol, narcotics (addiction,), substance craving and substance abuse; and disorders induced by chemical compounds, such as, substance intoxication, abstinency or withdrawal symptoms, substance-induced delirium, substance-induced persisting dementia, substance-induced persisting amnesic disorder, substance-induced psychotic disorder, substance-induced mood disorder, substance-induced anxiety disorder, substance-induced sexual dysfunction, substance-induced sleep disorder, substance-induced persisting perception disorder, flashbacks.
**"Receptors"** (from Latin *recipere*) represent biological macromolecules located either on cytoplasm membrane of the cell or intracellular, capable specifically interact with restricted number of physiologically active compounds (ligands) and transform the signal of this interaction into definite cellular response.
**"Psychic disorders, (psychic diseases)"** are diseases or diseased states associated with mental disturbance and/or mentality frustration. "Psychic disorders" include affective disorders (bipolar affective disorders, big depression, hypomania, minor depression, maniacal syndrome, Cotard's syndrome, cyclothymia, schizoaffective disorders and so on), intellectual-mnestic disorders; manias (hypomania, graphomania, kleptomania, compulsive shopping, mania of persecution, pornographomania, erotomania and so on); disorder of multiple personality, amentia, alcoholomania, deliration, delirium syndrome, hallucinosis, hallucinations, lucinatory effects, homicidomania, delirium; illusion, querulous paranoiaclinical lycanthropy, macropsia, antagonistic delusion, micropsia, narcomania; anorexia nervosa, oneiroid syndrome, paranoid, paranoia, paraphrenia, pseudohallucinations, psychosis, Cotard's syndrome, cyclothymia, schizoaffective disorder, intellectual-mnestic disorders, manias (hypomania, graphomania, kleptomania, compulsive shopping, persecution complex, monomania, pornographomania, erotomania and others), multiple personality disorder, amentia, delirium tremens, deliration, delirium syndrome, hallucinosis, hallucinations, hallucinosis, homicidomania, delirium, illusion, querulous paranoia, clinical lycathropy, antagonistic delusion, micropsia, narcomania, anorexia nervosa, oneiroid, paranoid, paranoia, paraphrenia, pseudohallucinations, psychosis, schizotypical disorder, schizophrenia, schizoaffective psychosis disorder, schizophrenomorphic disorder, Shrebera's syndrome, Daniel Paul's syndrome), phobias (agarophobia, arachnophobia, autophobia, verminophobia, hydrosophobia, hydrophobia, demophobia, zoophobia, carcinophobia, claustrophobia, climacophobia, xenophobia, misophobia, radiophobia, photophobia; skoliephobia, scotophobia, social phobia, tetraphobia, triskaidekaphobia, erotophobia); alcoholic psychosis, alcoholic palimpsest, allotriophagy, aphasia, graphomania, dissociative fugue state, dissociative disorders; dysphorias, internet-dependences, hypochondria, hysteria, kopophobia, delirium of persecution, melancholy, misanthropy, obsession, panic attacks, Asperger's syndrome, Capgras' syndrome, Munchausen's syndrome, Retta's syndrome, Fregoly's syndrome, syndrome of attention and hyperactivity deficit, obsessive-compulsive disorder, syndrome of chronic narcotization consequences, syndrome of psychic automatism, syndrome of infantile autism, madness, taphophilia, anxiety conditions, Hikikomory's syndrome, erotographomania and so on.
**"Psychotic diseases"** are all types of schizophrenia; schizoaffective psychosis; schizotypical disorders; schizoaffective disorders, including bipolar and depressive types; delirious disorders including reference delusion, delusion of persecution, megalomania, delusion of jealousy, erotomania, and also hypochondriacal, somatic, mixed and not differentiated delirium; short-time psychotic disorders; induced psychotic frustration; induced by substances psychotic frustration; and other psychotic disorders.
**"Therapeutic cocktail"** is simultaneously administered combination of two or more drug substances with different mechanisms of pharmacological action and aimed at different biotargets taking part in pathogenesis of the disease.
**"Anxiety disorders"** means generalized (inconcrete) anxiety; acute uncontrolled anxiety; panic disorder; phobia, for example, agoraphobia (acute fear of crowded places) or social (acute fear of humiliation in the presence of other people) or any other phobia (acute fear of particular subjects, animals or situations, in the form of phobia of height, of medical procedures, lifts, open space etc.); an obsessional condition (obsessive-compulsive disorder); posttraumatic stress disorder and acute stress disorder. Besides, anxiety disorders include anxiety conditions induced by alcohol or substances; anxiety under adaptation; as well as mixed forms of anxiety disorders and depression.
**"Schizophrenia"** means all known types, forms and variants of the disease, including: simple, hebephrenic, paranoid, hypertoxic (pyretic), catatonic, schizoaffective, residual or not differentiated schizophrenia and/or the forms of schizophrenia defined in classification of American Psychiatric Association (*American Psychiatric Association; in: Diagnostic and Statistical Manual of Mental Disorders,* IV Edition, Washington D.C. 2000) or in International classification (*International Statistical Classification of Diseases and Related Health Problems*) or any other known forms.
**"Pharmaceutical formulation"** means composition comprising, at least, one of the compounds of the general formula **1** and, at least, one of the components selected from pharmaceutically acceptable and pharmacologically compatible fillers, solvents, diluents, auxiliaries, distributing and sensing agents, delivery agents, such as preservatives, stabilizers, disintegrators, moisteners, emulsifiers, suspending agents, thickeners, sweeteners, flavoring agents, aromatizing agents, antibacterial agents, fungicides, lubricants, and prolonged delivery controllers, the choice and suitable proportions of which depend on the nature and way of administration and dose. Examples of suitable suspending agents are: ethoxylated isostearyl alcohol, polyoxyethene, sorbitol and sorbitol ether, microcrystalline cellulose, aluminum metahydroxide, bentonite, agar-agar and tragacant and their mixtures as well. Protection against microorganism action can be provided by various antibacterial and antifungal agents, such as: parabens, chlorobutanol, sorbic acid, and similar compounds. Composition may also contain isotonic agents, such as: sugar, sodium chloride, and similar compounds. Prolonged effect of the composition may be achieved by the agents inhibiting absorption of the active ingredient, for example, aluminum monostearate and gelatin. Examples of suitable carriers, solvents, diluents and delivery agents include water, ethanol, polyalcohols and their mixtures, natural oils (such as olive oil) and injection-grade organic esters (such as ethyl oleate). Examples of fillers are: lactose, milk-sugar, sodium citrate, calcium carbonate, calcium phosphate and the like. Examples of disintegrators and distributors are: starch, alginic acid and its salts, and silicates. Examples of suitable lubricants are: magnesium stearate, sodium lauryl sulfate, talc and polyethylene glycol of high molecular weight. Pharmaceutical formulation for peroral, sublingual, transdermal, intramuscular, intravenous, subcutaneous, local or rectal administration of active ingredient, alone or in combination with another active compound, may be administered to humans and animals in standard administration form, or mixture with traditional pharmaceutical carriers. Suitable standard administration forms include peroral forms such as tablets, gelatin capsules, pills, powders, granules, chewing-gums and peroral solutions or suspensions, for example, therapeutic cocktail; sublingual and transbuccal administration forms; aerosols; implants; local, transdermal, subcutaneous, intramuscular, intravenous, intranasal or intraocular forms and rectal administration forms.

The subject of the present invention is the novel ligand the range of pharmacological activity of which includes GPCR receptors, ion channels and monoamine transporters among them adrenoceptors, dopamine receptors, serotonin receptors, histamine receptors, sigma receptors, representing 3-methyl-9-benzyl-1,2,3,4-tetrahydrocarboline naphthalene-1,5-disulfonate of formula **1.**

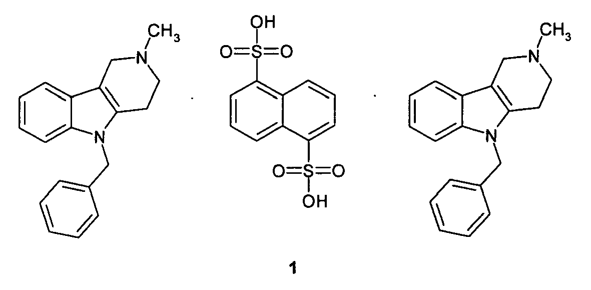

The subject of the present invention is the novel drug substance (active ingredient) with wide range of receptor-specific activity representing ligand of formula **1** for preparation of medicament intended for treatment and/or prophylaxis of CNS diseases, among them neurodegenerative diseases, cognitive, neuralgic and anxious disorders, psychic diseases of humans and warm-blooded animals.

The subject of the present invention is also a pharmaceutical formulation with wide range of receptor-specific activity for treatment and prophylaxis of CNS diseases, among them neurodegenerative diseases and cognitive disorders at humans and warm-blooded animals comprising pharmaceutically effective amount of the drug substance representing 3-methyl-9-benzyl-1,2,3,4-tetrahydrocarboline naphthalene-1,5-disulfonate of formula **1.**

Pharmaceutical formulations may include pharmaceutically acceptable excipients. Pharmaceutically acceptable excipients mean diluents, auxiliary agents and/or carriers applied in the sphere of pharmaceutics. Pharmaceutical formulation in addition to the active ingredient disclosed in this invention may include other active ingredients provided that, they do not give rise to undesirable effects, such as allergic reactions.

According to the present invention the carriers used in pharmaceutical formulations represent carriers, which are used in the sphere of pharmaceutics for preparation of commonly used forms. Binding agents, greasing agents, disintegrators, solvents, diluents, stabilizers, suspending agents, colorless agents, taste flavors are used for peroral forms; antiseptic agents, solubilizers, stabilizers are used in forms for injections; base materials, diluents, greasing agents, antiseptic agents are used in local forms.

The subject of the present invention is also a method for the preparation of pharmaceutical formulation which consists in mixing the active ingredient of formula 1 with inert exicipient and/or solvent.

The subject of the present invention is a medicament in the form of tablets, capsules, or injections, placed in pharmaceutically acceptable packing intended for prophylaxis and treatment of CNS diseases among them neurodegenerative diseases and cognitive, neuralgic and anxious disorders at humans and warm-blooded animals, comprising the novel drug substance or novel pharmaceutical formulation.

The preferable medicament comprising the novel drug substance or novel pharmaceutical formulation is the medicament intended for prophylaxis and/or treatment of Alzheimer's disease, Parkinson's disease, Huntington's disease (chorea), multiple sclerosis, cerebellar degeneration; amyotrophic lateral sclerosis; Lewy body dementia; Aran-Duchenne disease; peripheral neuropathy; bovine spongiform encephalopathy, AIDS-associated dementia, multi-infarct dementia; frontotemporal dementia; leukoencephalopathy, chronical neurodegenerative diseases; insult; ischemic, reperfusion and anoxic brain damage; epilepsia; cerebral ischaemia; glaucoma; traumatic brain injury; Down's syndrome; encephalomyelitis; meningitis; encephalitis; neuroblastoma; schizophrenia; depression; pathological states and disorders, appearing at hypoxia, excessive use of addictive substances at neurotoxin action; infectious and oncological cerebropathies; and also at neuronal damages associated with autoimmune and endocrine diseases.

The preferable medicament comprising the novel drug substance or the novel pharmaceutical formulation is a medicament intended for prophylaxis and/or treatment of mental capacity disorder including attention, memory, mentality, cognition, training, verbal, brain, dutiful and creative ability, orientation in time and space; senile dementia; age-associated memory impairment, AAMI; dismetabolic encephalopathy; psychogenic memory impairment; amnesia; amnestic disorders; transitory global amnesia; dissociative amnesia; vascular dementia; mild cognitive impairment, MCI; attention deficit hyperactivity disorder, AD/HD; cognitive disorders going along with psychotic diseases, epilepsy, delirium, autism, psychosis, Down's syndrome, bipolar disorders and depression; dementia at hypothyroidism; dementia induced by alcohol, by addictive substances and neurotoxins; dementia accompanying neurodegenerative diseases, for example, cerebellar degeneration and amyotrophic lateral sclerosis; cognitive disorders, taking place at insult, infectious and oncological cerebropathies, and also at traumatic brain injury; and also at cognitive disorders associated with autoimmune and endocrine diseases.

The subject of the present invention is a medicament (anxiolytic or tranquillizer) comprising the novel drug substance or novel pharmaceutical formulation intended for prophylaxis and treatment of anxious disorders.

The subject of the present invention is a medicament (nootropic) comprising the novel drug substance or novel pharmaceutical formulation intended for mental ability enhancement.

The subject of the present invention is a medicament comprising the novel drug substance or novel pharmaceutical formulation intended for treatment of schizophrenia.

The preferable medicament comprising the novel drug substance or the novel pharmaceutical formulation is a medicament intended for treatment of Alzheimer's disease.

The preferable medicament comprising the novel drug substance or the novel pharmaceutical formulation is a medicament intended for treatment of Parkinson's disease.

The preferable medicament comprising the novel drug substance or the novel pharmaceutical formulation is a medicament intended for treatment of Huntington's disease.

The subject of the present invention is also a therapeutic cocktail intended for prophylaxis and treatment of various diseases, pathogenesis of which is associated with adrenoceptors, dopamine receptors, serotonin receptors, histamine receptors, sigma receptors, monoamine transporters and ion cannels at humans and animals, including the novel drug substance or pharmaceutical formulation, comprising as drug substance 3-methyl-9-benzyl-1,2,3,4-tetrahydrocarboline naphthalene-1,5-disulfonate of formula **1.**

The subject of the present invention is also a therapeutic cocktail intended for prophylaxis and treatment of neuralgic disorders, neurodegenerative and cognitive diseases at humans and animals, among them for prophylaxis and treatment of Alzheimer's disease, Huntington disease, psychotic disorders and schizophrenia, hypoxia-ischemia, hypoglycemia, convulsive states, brain injuries, lathyrism, amyotrophic lateral sclerosis, obesity or insult, including the novel drug substance or pharmaceutical formulation comprising as drug substance 3-methyl-9-benzyl-1,2,3,4-tetrahydrocarboline naphthalene-1,5-disulfonate of formula **1.**

The therapeutic cocktail for prophylaxis and treatment of neuralgic and cognitive disorders and neurodegenerative diseases at humans and animals, among them for prophylaxis and treatment of Alzheimer's disease, Huntington's disease, psychotic disorders, schizophrenia, hypoxia-ischemia, hypoglycemia, convulsive states, brain injuries, lathyrism, amyotrophic lateral sclerosis and insult in addition to the drug substance disclosed in the invention, may include other active ingredients such as: nonsteroidal anti-inflammatory drugs (Orthophene, Indomethacin, Ibuprophen and others); acetyl cholinesterase inhibitors (Tacrine, Amiridine, Fizostigmine, Aricept, Phenserine and others); estrogens (for example, Estradiol); NMDA-receptor antagonists (for example, Memantine, Neramexane); nootropic drugs (for example, Pyracetam, Fenibut and others); AMPA receptor modulators (for example, Ampalex); cannabinoid CB-1 receptor antagonists (for example, Rimonabant); monoaminooxidase inhibitors MAO-B and/or MAO-A (for example, Rasagiline); antiamyloidogenic drugs (for example, Tramiprosate); lowering β-amyloidal neurotoxicity compounds (for example, Indole-3-propionic acid); γ- and/or β-secretase inhibitors; muscarinic receptor agonists (for example, Cevimeline); metal helates (for example, Clioquinol); GABA(A) receptor antagonists (for example, CGP-36742); monoclonal antibodies (for example, Bapineuzumab); antioxidants; neurotrophic agents (for example, Cerebrolisine); antidepressants (for example, Imipramine, Sertraline and others) and others.

According to the invention a method for prophylaxis and treatment of various CNS diseases, among them neurodegenerative diseases and cognitive disorders at humans and animals consists in introduction of a medicament in the form of tablets, capsules, or injections, or pharmaceutical formulation, comprising the novel drug substance, or the drug substance representing ligand of formula **1** in effective amount.

Medicaments could be introduced peroral or parenterally (for example, intravenously, subdermally, intraperitoneally or locally). Clinical dose of a drug substance of formula **1** could be corrected depending on: therapeutic efficiency and bio-accessibility of active ingredients in patients' organisms, rate of their exchange and removal from organism, and age, gender, and severity of patient's symptoms. Thus, the daily intake for adults is normally being 10 ~ 500 mg, preferably 10 ~ 300 mg. Therefore the above effective doses are to be taken into consideration while preparing pharmaceutical formulations in the form of dose unit according to the present invention; each dose unit of medicament should contain 10 ~ 500 mg, preferably - 50 ~300 mg. Following the instructions of physician or pharmacist, the medicaments may be taken several times over specified periods of time (preferably, from one to six times).

### Best Embodiment of the invention

The invention is illustrated by the following figures.

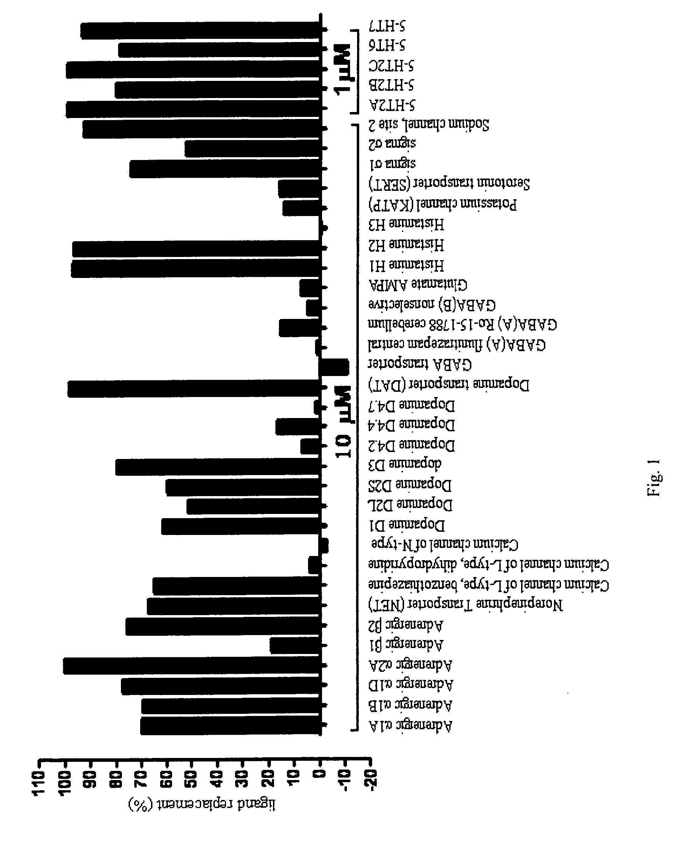

**Fig. 1** Profile of pharmacological activity of 3-methyl-9-benzyl-1,2,3,4-tetrahydrocarboline naphthalene-1,5-disulfonate of formula **1** (10 µM) on the panel of therapeutic targets (GPCR receptors, ion channels and monoamine transporters) in the setting of radioligand binding.

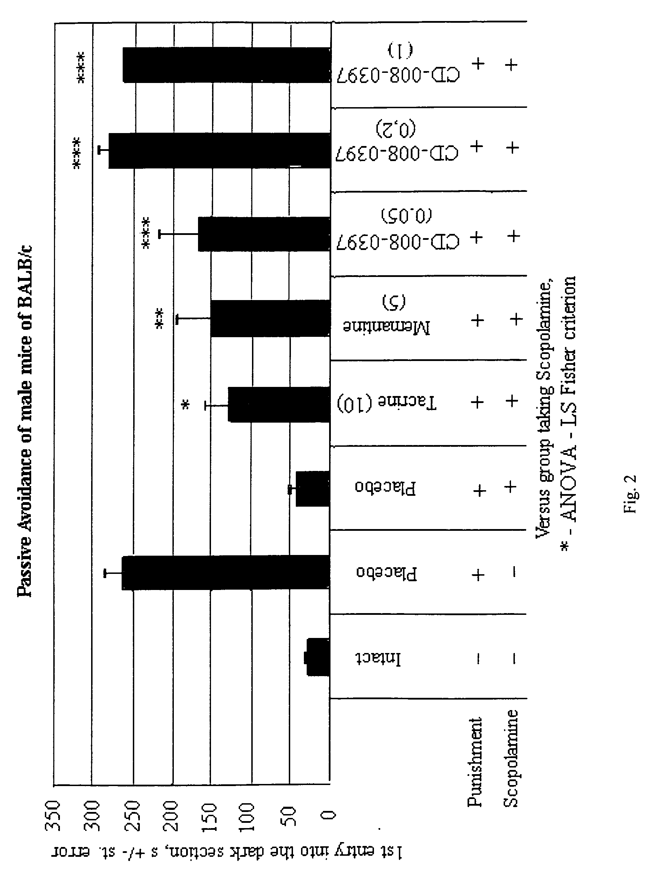

**Fig. 2** Enhancement of memory disturbed by Scopolamine in male mice of BALB/c line, under the influence of drug substance of formula **1** (CD-008-0397) and reference substances (Tacrine and Memantine) in the test "Passive Avoidance of mice in the Shuttle Chamber". Latent period of the first entry into the dark chamber. The concentration of the drug substance in mg/kg is given in brackets.

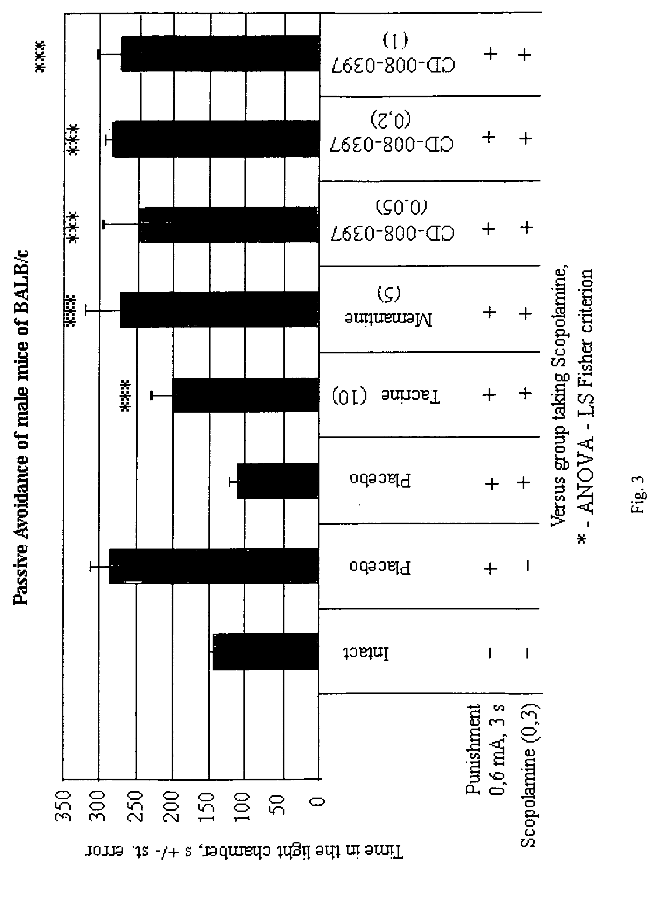

**Fig. 3** Enhancement of memory disturbed by Scopolamine in male mice of BALB/c line, under the influence of drug substance of formula **1** (CD-008-0397) and reference substances (Tacrine and Memantine) in the test "Passive Avoidance of mice in the Shuttle Chamber". The time spent by the animals in the light chamber. The concentration of the drug substance in mg/kg is given in brackets.

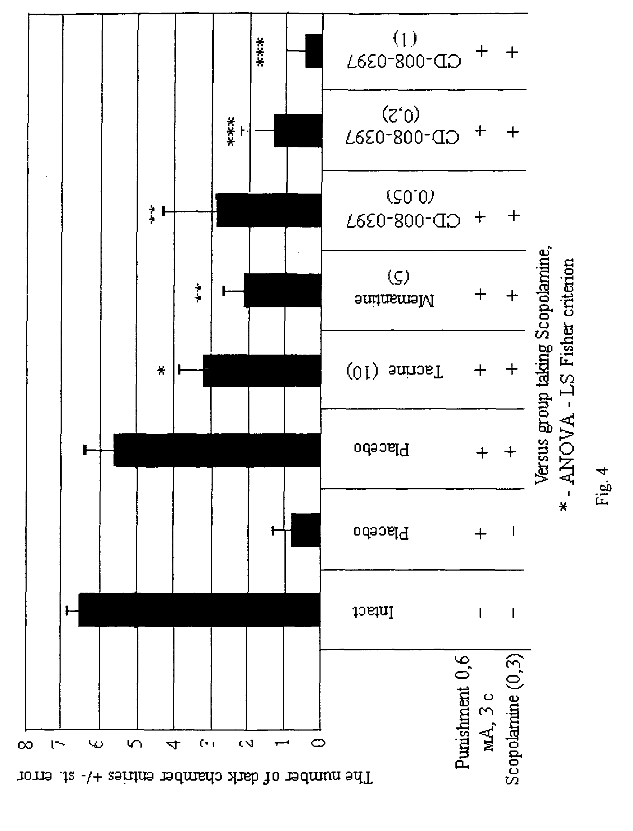

**Fig. 4** Enhancement of memory disturbed by Scopolamine in male mice of BALB/c line, under the influence of drug substance of formula **1** (CD-008-0397) and reference substances (Tacrine and Memantine) in the test "Passive Avoidance of mice in the Shuttle Chamber". The number of dark chamber entries. The concentration of the drug substance in mg/kg is given in brackets.

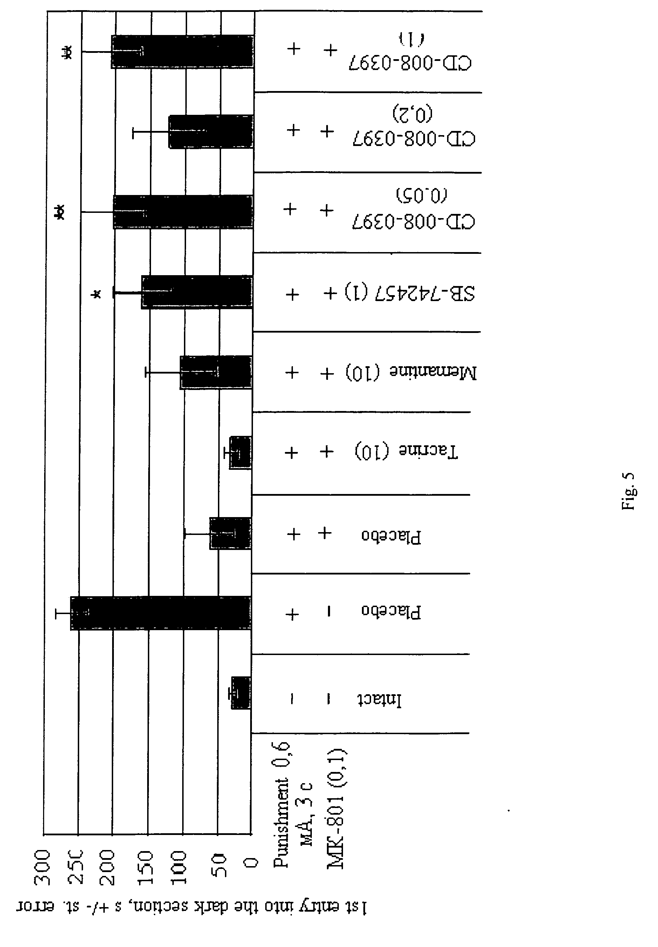

**Fig. 5** Enhancement of memory disturbed by MK-801 in male mice of BALB/c line, under the influence of drug substance of formula **1** (CD-008-0397) and reference substances (Tacrine and Memantine) in the test "Passive Avoidance of mice in the Shuttle Chamber". Latent period of the first entry into the dark chamber. The concentration of the drug substance in mg/kg is given in brackets.

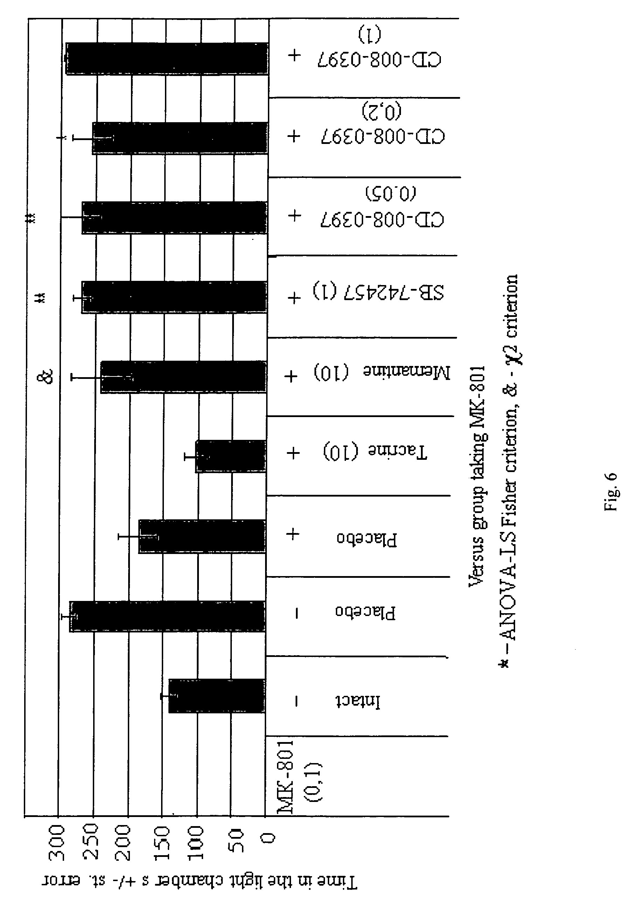

**Fig. 6** Enhancement of memory disturbed by MK-801 in male mice of BALB/c line, under the influence of drug substance of formula **1** (CD-008-0397) and reference substances (Tacrine and Memantine) in the test "Passive Avoidance of mice in the Shuttle Chamber". The time spent by the animals in the light chamber. The concentration of the drug substance in mg/kg is given in brackets.

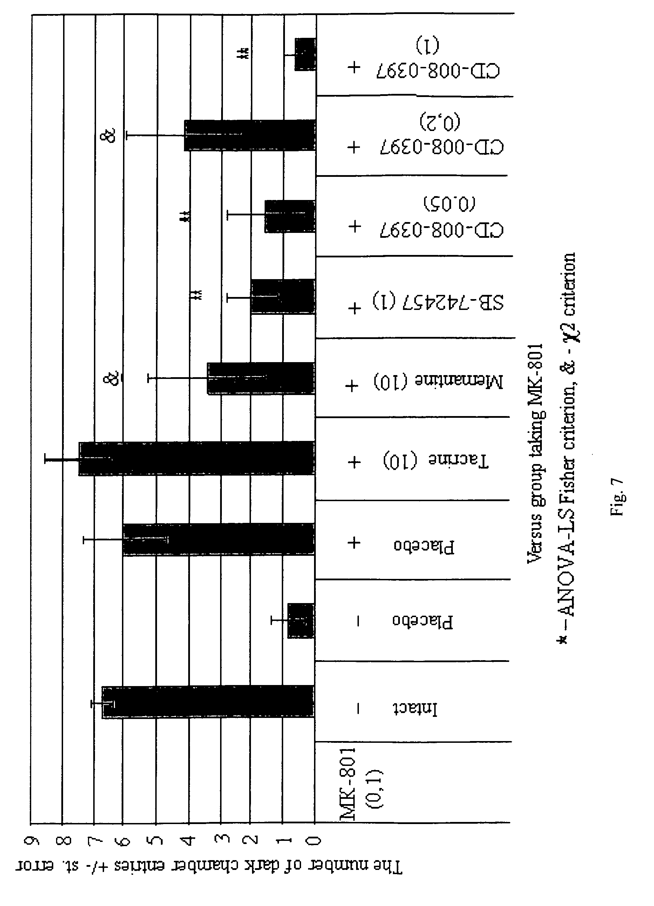

**Fig. 7** Enhancement of memory disturbed by MK-801 in male mice of BALB/c line, under the influence of drug substance of formula **1** (CD-008-0397) and reference substances (Tacrine and Memantine) in the test "Passive Avoidance of mice in the Shuttle Chamber". The number of dark chamber entries. The concentration of the drug substance in mg/kg is given in brackets.

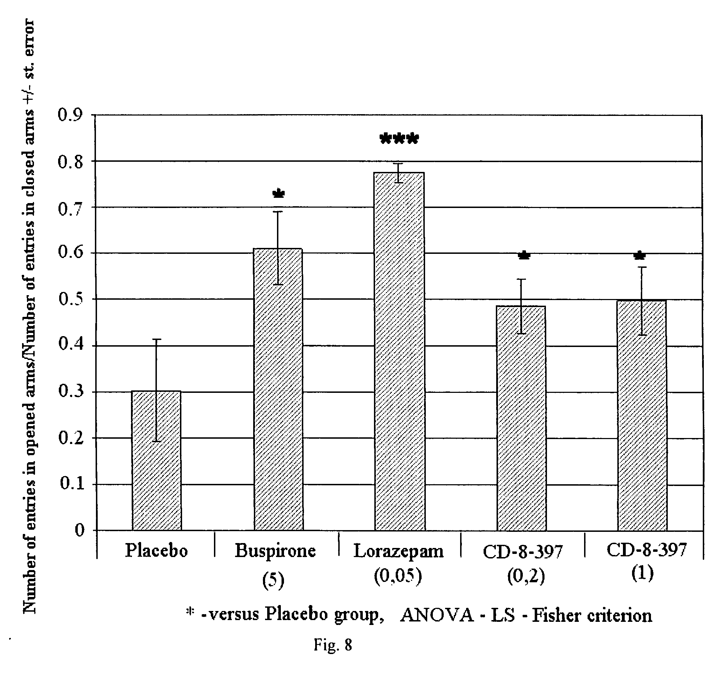

**Fig. 8** Behavior of male mice of BALB/c line under the influence of drug substance of formula 1 (CD-008-0397) and reference substances (Buspirone and Lorazepam) in the test "Mice Behavior in the Elevated Plus Maze". The number of open arms entries. The concentration of the drug substance in mg/kg is given in brackets.

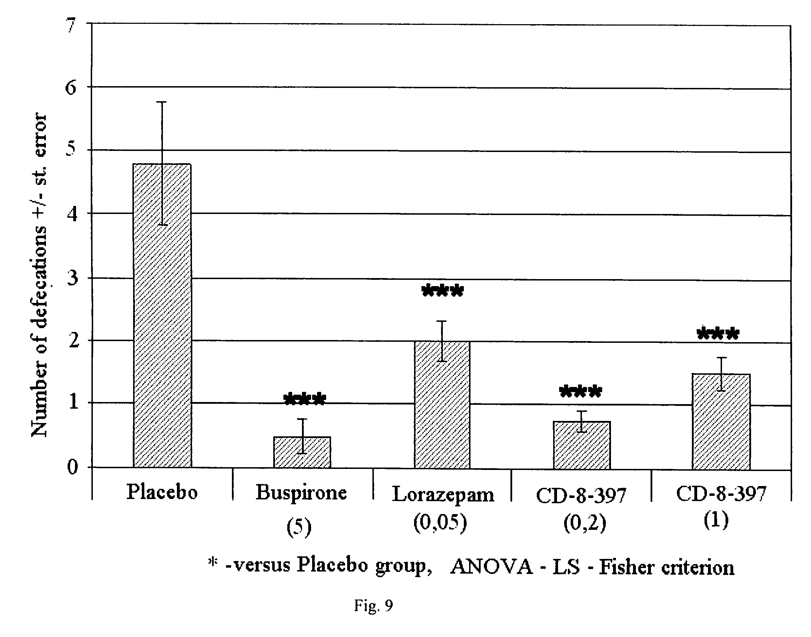

**Fig. 9** Behavior of male mice of BALB/c line BALB/c under the influence of drug substance of formula **1** (CD-008-0397) and reference substances (Buspirone and Lorazepam) in the test "Mice Behavior in the Elevated Plus Maze". The number of defecations. The concentration of the drug substance in mg/kg is given in brackets.

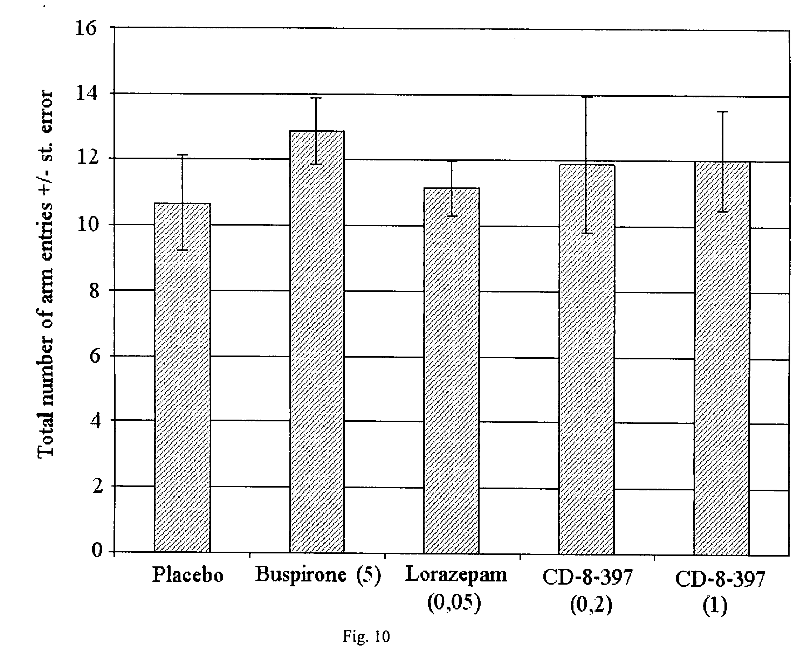

**Fig. 10** Behavior of male mice of BALB/c line under the influence of drug substance of formula **1** (CD-008-0397) and reference substances (Buspirone and Lorazepam) in the test "Mice Behavior in the Elevated Plus Maze". The total number of arm entries. The concentration of the drug substance in mg/kg is given in brackets.

Below the invention is described by means of specific examples, which illustrate but not limit the scope of the invention.

**Example 1.** The determination of pharmacological activity profile of the disclosed compound of formula **1** on the panel of potential therapeutic targets including GPCR receptors, ion channels and monoamine transporters, and other. The firm MDS Pharma Services [http://www.mdsps.com] carried out this test in duplicates at ligand concentration 10µM by the displacement of the corresponding radioactive-labeled ligand. Test results, expressed in percent of ligand replacement (%), represented in Table 2 and in Fig. 1.

**Table 2. Test results for pharmacological activity of ligand of formula 1 on the panel of molecular therapeutic targets.**

| N° | Target | Source | % of radioactive-labeled ligand replacement |
|---|---|---|---|
| 1 | adrenergic α_{1A} | rat | 70 |
| 2 | adrenergic α_{1B} | rat | 70 |
| 3 | adrenergic α_{1D} | human being | 77 |
| 4 | adrenergic α_{2A} | human being | 100 |
| 5 | adrenergic β₂ | human being | 76 |
| 6 | calcium channel of L-type, benzothiazepine | rat | 65 |
| 7 | dopamine D₁ | human being | 62 |
| 8 | dopamine D_{2L} | human being | 51 |
| 9 | dopamine D_{2S} | human being | 60 |
| 10 | dopamine D₃ | human being | 80 |
| 11 | histamine H₁ | human being | 97 |
| 12 | histamine H₂ | human being | 97 |
| 13 | serotonin 5-HT_{1B} | human being | 64* |
| 14 | serotonin 5-HT_{2A} | human being | 68 |
| 15 | serotonin 5-HT_{2B} | human being | 52 |
| 16 | serotonin 5-HT_{2C} | human being | 79 |
| 17 | serotonin 5-HT₆ | human being | 79 |
| 18 | serotonin 5-HT₇ | human being | 76 |
| 19 | sigma σ₁ | human being | 75 |
| 20 | sigma σ₂ | rat | 52 |
| 21 | sodium channel, site 2 | rat | 93 |
| 22 | dopamine transporter (DAT) | human being | 99 |
| 23 | norepinephrine transporter (NET) | human being | 67 |

Test results (Table 2, fig. 1) testify that ligand of formula **1** exhibits a wide range of pharmacological activity and interacts with the great number of previously unaccounted targets, inter alia adrenergetic, dopamine, serotonin and other receptors, ion channels and monoamine trasmitters.

**Example 2.** The preparation of medicament in the form of tablets. Starch (1600 mg), ground lactose (1600 mg), talk (400 mg) and 3-methyl-9-benzyl-1,2,3,4-tetrahydrocarboline naphthalene-1,5-disulfonate of formula **1** (1000 mg) were mixed together and pressed into a bar. The resultant bar was comminuted into granules and sifted through sieve to collect granules of 14-16 mesh. The granules thus obtained were shaped into tablets of suitable form weighing 560 mg each.

**Example 3.** The preparation of medicament in the form of capsules. 3-Methyl-9-benzyl-1,2,3,4-tetrahydrocarboline naphthalene-1,5-disulfonate of formula **1** and lactose powder were carefully mixed in ratio 2 : 1. The resultant powdery mixture was packed into gelatin capsules of suitable size by 300 mg to capsule.

**Example 4.** The preparation of medicament in the form of injectable compositions for intramuscular, intraperitoneal or hypodermic injections. 3-Methyl-9-benzyl-1,2,3,4-tetrahydrocarboline naphthalene-1,5-disulfonate of formula **1** (500 mg), chlorobutanol (300 mg), propylene glycol (2 ml) and injectable water (100 ml) were mixed together. The resultant solution was filtered and placed into 1 ml ampoules, which were sealed and sterilized in autoclave.

**Example 5.** Nootropic action of (enhancement of memory disturbed by Scopolamine) drug substace of formula 1 in the test "Passive Avoidance of mice in the Shuttle Chamber".

A shuttle chamber (Ugo Basile, Italy) that consisted of two sections was used. One section had all the walls opaque, the other had transparent cover. The sections were connected through a hole which could be overlapped by automatic vertical door. The floor of the dark section was made of transverse metal bars on which DC current impulses could be fed. The experiments were carried out in aged male mice of BALB/c line weighing 20-25g.

On the first day of testing 30 minutes before training the animals were injected intraperitoneally with physiological solution, Scopolamine (0.3 mg/kg) or Scopolamine in combination with 3-methyl-9-benzyl-1,2,3,4-tetrahydrocarboline naphthalene-1,5-disulfonate of formula **1.** Each group consisted of 8 animals.

The animals were placed in the light section, and latent period of the first entry into the dark chamber was registered. Then the vertical door was closed and the animal was punished by 0.6 mA DC current for 3 seconds. After that the animal was taken back to its home cage. In 22-24 hours the same animal was placed again in the light section of the shuttle chamber and latent period of its first entry into the dark section, the total time of its stay in the light section and the number of entries into the dark section were registered. Each monitoring lasted for 5 minutes.

The experiments were carried out during the light period of animal's diurnal in isolated laboratory using "white noise" of about 70 dB above threshold of human hearing.

The memory training is expressed in prolongation of latent period of the first entry into the dark section, increasing the time of stay in the light section and decreasing the number of entries into the dark section. Scopolamine causes training disturbance (memory disturbance).

The ability of 3-methyl-9-benzyl-1,2,3,4-tetrahydrocarboline naphthalene-1,5-disulfonate of formula **1** to improve training disturbed by Scopolamine is considered as the evidence of its nootropic action.

The test results presented in fig. 2, 3 and 4, testify the ability of 3-methyl-9-benzyl-1,2,3,4-tetrahydrocarboline naphthalene-1,5-disulfonate of formula **1** to produce nootropic action.

**Example 6.** Nootropic action of (enhancement of memory disturbed by MK-801) drug substance of formula 1 in the test "Passive Avoidance of mice in the Shuttle Chamber".

The test was carried out as in example 5. On the first day of the test 30 minutes before training the mice were injected intraperitoneally with physiological solution of MK-801 (0.1 mg/kg). In parallel, physiological solution of MK-801 in combination with 3-methyl-9-benzyl-1,2,3,4-tetrahydrocarboline naphthalene-1,5-disulfonate of formula **1** was injected intraperitoneally to independent groups of mice before training.

The test results represented in fig. 5-7, testify the ability of 3-methyl-9-benzyl-1,2,3,4-tetrahydrocarboline naphthalene-1,5-disulfonate of formula **1** to produce nootropic action.

**Example 7.** Anxiolytic (tranquilizing) action of the drug substance of formula **1** in test "Mice Behavior in the Elevated Plus Maze".

The length of maze arms makes 30cm, the width 5cm, height of walls 15cm. Two opposite arms are closed from sides and end walls with prasparent walls, the two others are lighted and opened. A mouse was placed in the maze center and during 5 minutes the number of opened and closed arm entries and the time spent by the animals in opened and closed arms were registered. On this evidence indexes of opened arms preference were calculated as a ratio of the number of opened arm entries and also duration of their stay in the opened arms to the total number of entries into all of arms and duration of stay in them. Being in normal state animals avoid opened arms (index of preference makes 0.2-0.3). Compounds with anxiolytic activity (tranquilizing activity) run this value up to 0.5-0.6 and more, and also decrease the number of defecations without changing overall physical activity (general number of arm entries).

The test result (fig. 8-10) testify that drug substance of formula **1** exhibibts anxiolytic (tranquilizing) activity comparable with the activity of Buspirone and Lorazepam

### Industrial applicability

The invention could be used in medicine, veterinary, biochemistry

## Claims

1. Use of 3-methyl-9-benzyl-1,2,3,4-tetrahydrocarboline naphthalene-1,5-disulfonate of formula **1** as a ligand with the wide range of pharmacological activity.

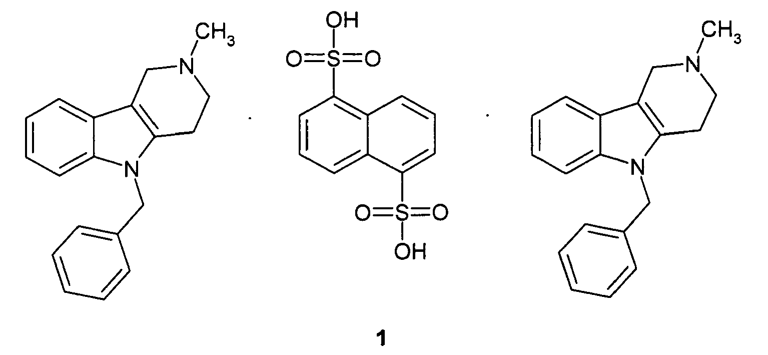

2. Use according to claim 1 as a ligand exhibiting activity to adrenergic receptors.

3. Use according to claim 1 as a ligand exhibiting activity to dopamine receptors.

4. Use according to claim 1 as a ligand exhibiting activity to serotonin receptors.

5. Use according to claim 1 as a drug substance for medicaments intended for prophylaxis and/or treatment of CNS diseases pathogenesis of which is associated with GPCR receptors, ion channels and monoamine transporters.

6. Pharmaceutical formulation with the wide range of pharmacological activity comprising pharmaceutically effective amount of the drug substance according to claim 5.

7. Method for the preparation of the pharmaceutical formulation according to claim 6 by mixing the drug substance according to claim 5 with inert filler and/or solvent.

8. A medicament in the form of tablets, capsules, or injections placed in pharmaceutically acceptable packing comprising the drug substance according to claim 5 or Pharmaceutical formulation according to claim 6 intended for prophylaxis and treatment of CNS diseases pathogenesis of which is associated with GPCR receptors, ion channels and monoamine transporters.

9. The medicament according to claim 8 for treatment of heurodegenerative diseases and cognitive disorders.

10. The medicament according to claim 8 for treatment of Alzheimer's disease.

11. The medicament according to claim 8 for treatment of Parkinson's disease.

12. The medicament according to claim 8 for treatment of Huntington's disease.

13. The medicament according to claim 8 for treatment of schizophrenia.

14. The medicament (anxiolytic or tranquillizer) according to claim 8 for prophylaxis and treatment of anxiety disorders.

15. The medicament (nootropic) according to claim 8 for enhancement of mental ability.

16. A method for prophylaxis and/or treatment of CNS diseases, pathogenesis of which is associated with GPCR receptors, ion channels and monoamine transporters by introduction of the drug substance according to claim 5, or pharmaceutical formulation according to claim 6, or medicament according to any of claims 8-15 to human being or warm-blooded animal.
